Europäisches Patentamt

European Patent Office

Office européen des brevets

(11) Publication number: **0 326 635 B1**

# (12) EUROPEAN PATENT SPECIFICATION

(45) Date of publication of patent specification: **22.07.92**   (51) Int. Cl.⁵: **C12Q 1/10, C07D 311/16**

(21) Application number: **88101566.3**

(22) Date of filing: **04.02.88**

(54) **Method for the immediate identification of salmonella cultures.**

(43) Date of publication of application:
**09.08.89 Bulletin 89/32**

(45) Publication of the grant of the patent:
**22.07.92 Bulletin 92/30**

(84) Designated Contracting States:
**AT BE CH DE ES FR GB GR LI NL SE**

(56) References cited:
**EP-A- 0 091 837**
**GB-A- 2 050 418**
**US-A- 3 553 235**

**ORGANIC CHEMISTRY, vol. 1, 4th edition,
Longmans, Green and Co. Ltd., London (GB),
1963; I.L.FINAR, p. 190**

(73) Proprietor: **BIOLIFE ITALIANA S.R.L.**
**Viale Monza, 272**
**I-20128 Milan(IT)**

(72) Inventor: **Bottiroli, Ugo**
**Via Losanna, 44**
**I-20154 Milan(IT)**
Inventor: **Carozzi, Fiorenzo**
**Via Sacco e Vanzetti, 5/1**
**I-21040 Carnago, VA(IT)**

(74) Representative: **Giambrocono, Alfonso, Dr.**
**Ing. et al**
**Ing. A. Giambrocono & C. S.r.l. Via Rosolino**
**Pilo 19/B**
**I-20129 Milano(IT)**

EP 0 326 635 B1

**Description**

The present invention concerns a method for identifying immediately Salmonella cultures and for distinguishing them from other enterobacteria.

Salmonellae represent the most common cause of intestinal bacterial infection.

The infection is characterized by an oro-fecal transmission and can be produced by either the ingestion of contaminated food or the contact with carriers, man or animal.

The identification of Salmonellae is one of the most important diagnostic tests which are carried out for both prevention and diagnosis purposes and the fast response and the simple operative procedure are the essential and requisite qualifications of these methods.

In fact, the most serious diagnostic problem in the infections by Salmonellae is represented by the time, presently very long, necessary for their definitive identification using the methods known so far.

The growth in liquid culture medium takes 12-18 hours, the isolation in solid culture medium 18 hours and the identification 4-18 hours depending on the selected method.

The total analysis time is in the range of 34 to 54 hours; including the times between the various analytical steps, the "actual" times amount to 2-3 days.

Such a situation brings about therapeutic drawbacks because, in the wait for the result of the investigation, the antibiotic therapy can not be specific, social drawbacks due to the exclusion of the suspected subject from the community and economic drawbacks for the several days long blocking of food stocks.

Object of the present invention is a novel, very easy, diagnostic method, which permits to reduce the times of the presumptive identification from the present 4-18 hours to 1-15 minutes.

The method consists in the identification of the esterase enzyme on characteristic growths of a nature medium selective for Salmonellae and enterobacteria or on any other cultural substrate showing a microbial growth, by employing a reagent containing the substrate specifically affected, under definite experimental condition, by the enzyme.

The reagent employed in the method according to the invention is composed by a carboxylic acid ester of 4-methylumbelliferone fluorophore dissolved in organic solvent, such as acetone, dimethylformamide, methylcellosolve, butylen glycol or other.

More particularly it has been found that a suitable reagent is the 4-methylumbelliferone ester of a carboxylic acid having 7-10 carbon atoms.

The bacteria enzyme present in the cultures hydrolizes the substrate and releases 4-methylumbelliferone, which is strongly fluorescent under Wood's lamp.

The test is easily carried out by depositing a drop of reagent on the microbial growth and observing the appearance of fluorescence within 1-5 minutes.

The method for identifying Salmonellae according to the present invention represents a remarkable advancement compared to the presently available methods, said method being in particular faster and easier that the known ones, such as the one described in French patent 2.457,323. According to the known method, the results are obtained after preferably 2-8 hours by carrying out culture treatments not contemplated by the present invention or after a shorter time, being necessary in this case to incorporate in the culture medium the substrate to be affected by the enzyme, which are complex and hardly feasible procedures in routine tests of a microbiology laboratory. Moreover, the method of the above-mentioned French patent differs from the present invention, because the identification of Salmonellae is based on a colorimetric reaction due to a diazonium salt, to be added to the reagent in every case.

The document EP-A-0 091 837 describes a method for establishing the microbial sensitivity to antibiotics by determination of the MIC (minimum inhibitory concentration) using, as indicators of microbial growth, umbelliferone derivatives.

The present process on the contrary, consists of an identification of Salmonella by using as a substrate umbelliferone derivatives.

A further advantage of the diagnostic method of the present invention is that the employed reagent allows to identify the germs Salmonella within 1-5 minutes, without carrying out the definitive identification of every culture having, on a selective solid medium, characteristics similar to Salmonellae (f.i. Proteus), therefore saving time and materials.

From experiments carried out on 572 Salmonella stocks according to the method of the invention, it has been found that the sensitivity of the reagent in ascertaining the bacterial presence is 95,4%, its specificity being 90,8%.

The method of identification of Salmonellae according to the invention works even by placing the growths on paper disks or cotton pads impregnated with the reagent object of the present invention, instead of placing this latter on the growths of the culture medium.

The acyl esters of 4-methylymbelliferone are known compounds, described in U.S. Patent 3.553.235 and their use as fluorogenic substrates in determining the carboxylesterase and, more particularly, lipase activities is also known.

A preferred method for preparing the 4-methylumbelliferone derivatives consists in preparing a solution of 4-methylumbelliferone, preferably in form of a sodium salt, in a solvent such as acetone or methylene chloride and treating said solution with a stechiometric amount, or a small excess of the chloride of the desired carboxylic acid having 7-10 carbon atoms. The reaction is carried out at room temperature and under anhydrous conditions and the product can be isolated and purified according to known procedures.

The invention is illustrative by the following examples which concern the preparation of 4-methylumbelliferone derivatives for illustrative purposes and the identification method of Salmonellae.

EXAMPLE 1

4-Methylumbelliferone heptanoate

2g of 4-methylumbelliferone, sodium salt, are dissolved in 20 ml of acetone; 4 g of heptanoyl chloride are added dropwise at room temperature and under anhydrous conditions. When, after a short time, the reaction is completed, the solvent is evaporated and the residue taken up in methylene chloride, while repeatedly washing with diluted sodium bicarbonate; the organic phase is separated, dried and the solvent evaporated under vacuum; the title compound, purified by crystallization from methanal/hezane - heptane - octane, is obtained in 95% yield, m.p. 39-41°C.

| Elementar analysis | | |
|---|---|---|
| Calculated | C 70,8% | H 6,99% |
| Found | C 71,0% | H 6,89% |

EXAMPLE 2

4-Methylumbelliferone octanoate

2 g of 4-methylumbelliferone, sodium salt, are dissolved in 20 ml of acetone and 4,0 g of octanoyl chloride are added dropwise at 20°C and under anhydrous conditions; when, after a short-time, the reaction is completed, the solvent is evaporated and the residue taken up in methylene chloride, while repeatedly washing with diluted sodium bicarbonate; the organic phase is separated, dried and the solvent evaporated under vacuum. The title compound, purified by crystallization from methanol/hexane-heptane-octane, is obtained in 97.5% yield, m.p. 41-43°C.

| Elemental analysis | | |
|---|---|---|
| Calculated | C 71,5% | H 7,33% |
| Found | C 71,6% | H 7,35% |

EXAMPLE 3

4-Methylumbelliferone nonanoate

2 g of 4-methylumbelliferone, sodium salt, are dissolved in 20 ml of acetone and 4 g of nonanoyl chloride are added dropwise at 18°C and under anhydrous conditions; when, after a short time, the reaction is completed, the solvent is evaporated and the residue taken up in methylene chloride, while repeatedly washing with diluted sodium bicarbonate; the organic phase is separated, dried and the solvent evaporated under vacuum. The title compound, purified by crystallization from methanol/hezane-heptane-octane, is obtained in 95% yield, m.p. 47-49°C.

| Elemental analysis | | |
| --- | --- | --- |
| Calculated | C 72,12% | H 7,64% |
| Found | C 72,2% | H 7,68% |

EXAMPLE 4

Identification method of Salmonella spp.

On a bed of SS Agar selective culture medium colonies of Salmonella typhimurium and Proteus mirabilis in mixed flora are cultured. Both cultures fail to ferment lactose and are $H_2S$-positive. A drop of 4-methylumbelliferone octanoate dissolved in acetone is deposited on the microbial growth. After 2-3 minutes a fluorescence under Wood's lamp at 366 nm is observed. The colonies of Salmonella typhimurium show a strong blue fluorescence, whereas the colonies of Proteus mirabilis do not show fluorescence.

It is therefore possible to distinguish Salmonella spp in mixed growth, which was by any other means impossible, with consequent obvious possible diagnostic error.

The term "SS Agar" as hereinabove used refers, in an abbreviated form which is familiar to those working in the field, to the culture media Salmonella Shigella Agar.

**Claims**

1. Method for identifying immediately Salmonellae and distinguishing them from other non fermenting, $H_2S$-positive or negative bacteria, characterized in that the method is carried out directly on the microbial culture medium by employing a reagent consisting of a 4-methylumbelliferone ester of a carboxylic acid with 7-10 carbon atoms, which is specifically affected by the bacterial enzyme present in the cultures, and observing the resulting fluorescence within 1-5 minutes.

2. Method according to claim 1, characterized in that the employed reagent is 4-methylumbelliferone octanoate.

3. Method according to claim 1, characterized in that paper disks or cotton pads are impregnated with the reagent and the microbial growth is deposited thereon.

**Revendications**

1. Procédé pour l'identification immédiate des Salmonelles et leur différenciation d'autres bactéries non fermentescibles, $H_2S$-positives ou négatives, caractérisé par ce que le procédé est exécuté directement sur le terrain de culture microbien en employant un réactif composé par un ester de la 4-méthyl-umbelliférone avec un acide carbonique ayant 7-10 atomes de carbone, qui est influencé spécifiquement par l'enzyme bactérien qui est présent dans les cultures, et en observant la fluoréscence qui se forme entre 1-5 minutes.

2. Procédé selon la revendication 1, caractérisé par ce que le réactif employé est octanoate de 4-méthylumbelliférone.

3. Procédé selon la revendication 1, caractérisé par ce que des disques de papier ou des tampons de coton sont imprégnés avec le réactif et la culture microbienne est déposée sur ces disques ou tampons.

**Patentansprüche**

1. Verfahren zur sofortigen Identifizierung von Salmonellae und deren Unterscheidung von anderen nicht gärenden, $H_2S$-positiven oder negativen Bakterien, dadurch gekennzeichnet, dass das Verfahren unmittelbar auf dem mikrobischen Kulturmedium ausgeführt wird mit Verwendung eines Reagens bestehend aus einem 4-Methylumbelliferonester einer 7-10 Kohlenstoffatome enthaltenden Carbonsäure, das mit dem in den Kulturen anwesenden bakteriellen Enzym spezifisch reagiert, und die entstehende Fluoreszenz binnen 1-5 Minuten bemerkt.

**2.** Verfahren nach Anspruch 1, dadurch gekennzeichnet, dass das verwendete Reagens 4-Methylumbelliferonoctansäureester ist.

**3.** Verfahren nach Anspruch 1, dadurch gekennzeichnet, dass Papierscheiben oder Wattebäusche mit dem Reagens imprägniert werden und die mikrobische Kultur darauf niederlegt wird.